(19) Europäisches Patentamt
European Patent Office
Office européen des brevets

(11) Veröffentlichungsnummer: **0 292 911**
**A2**

(12) # EUROPÄISCHE PATENTANMELDUNG

(21) Anmeldenummer: **88108213.5**

(51) Int. Cl.⁴: **C07C 85/26 , C07C 87/60**

(22) Anmeldetag: **21.05.88**

(30) Priorität: **27.05.87 DE 3717833**

(43) Veröffentlichungstag der Anmeldung:
**30.11.88 Patentblatt 88/48**

(84) Benannte Vertragsstaaten:
**CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Tronich, Wolfgang, Dr.**
**Adolf-Guckes-Weg 5**
**D-6239 Eppstein/Taunus(DE)**
Erfinder: **Wykypiel, Werner, Dr.**
**Egerstrasse 7**
**D-6054 Rodgau(DE)**

(54) **Verfahren zur Herstellung von reinen Nitro-aminobenzol-Verbindungen.**

(57) Nitro-aminobenzol-Verbindungen, die durch übliche Nitrierung einer an der Aminogruppe mit einer Schutzgruppe versehenen Aminobenzol-Verbindung erhältlich sind, werden rein hergestellt, indem man die aminogruppengeschützte Nitro-aminobenzol-Verbindung zunächst isoliert, die Schutzgruppe mittels Schwefelsäure abspaltet, sodann das aus der Hydrolyse erhaltene Sulfatsalz der Nitro-aminobenzol-Verbindung in Form des reinen, kristallinen Sulfates aus der Reaktionsmischung abscheidet und isoliert und hieraus gewünschtenfalls die freie Nitro-aminobenzol-Verbindung in reiner Form gewinnt.

EP 0 292 911 A2

## Verfahren zur Herstellung von reinen Nitro-aminobenzol-Verbindungen

Die Erfindung liegt auf dem technischen Gebiet der Herstellung von Zwischenprodukten, die bevorzugt zur Synthese von Farbstoffen eingesetzt werden.

Es ist aus der Literatur bekannt, substituierte Nitro-aminobenzol-Verbindungen in der Weise herzustellen, daß man von einer an der Aminogruppe mit einer Schutzgruppe versehenen Aminobenzol-Verbindung ausgeht und diese in einem geeigneten Reaktionsmedium nitriert, anschließend die erhaltene aminogruppengeschützte Nitro-aminobenzol-Verbindung durch saure oder alkalische Abspaltung der Schutzgruppe in die freie Aminoverbindung überführt und diese aus einer stark verdünnten sauren oder alkalischen, wäßrigen Suspension isoliert. Im allgemeinen ist es sodann notwendig, die erhaltene Nitro-aminobenzol-Verbindung einer zusätzlichen Reinigung zu unterziehen, um sie in einer hinreichend guten Qualität weiterverarbeiten zu können.

So verfährt man nach einem üblichen Verfahren zur Herstellung von 5-Nitro-2-amino-1-methoxy-benzol (s. BIOS Final Report Nr. 986, S. 279) in der Weise, daß man das durch Nitrierung von 2-Acetylamino-1-methoxy-benzol in wäßriger Schwefelsäure erhaltene, aus 5-Nitro-2-acetylamino-1-methoxy-benzol und 4-Nitro-2-acetylamino-1-methoxy-benzol bestehende Isomerengemisch mittels wäßriger Schwefelsäure hydrolysiert und anschließend die Trennung der beiden isomeren Nitroamino-methoxy-benzol-Verbindungen in der Weise durchführt, daß man die Mineralsäure mit Natriumcarbonat abstumpft und den Ansatz mit Wasser stark verdünnt, wobei das 5-Nitro-2-amino-1-methoxy-benzol ausfällt und durch Filtration isoliert werden kann (aus der Mutterlauge läßt sich das 4-Nitro-2-amino-1-methoxy-benzol gewinnen). Das so erhaltene 5-Nitro-2-amino-1-methoxy-benzol ist jedoch bei weitem noch nicht genügend rein, um unter der Colour Index-Bezeichnung Azoic Diazo Compound 5 als Diazokomponente in der Eisfarbentechnik zur Herstellung von qualitativ guten Färbungen verwendet werden zu können. Vielmehr muß es zunächst durch eine aufwendige Umkristallisation aus Wasser unter Druck gereinigt werden (s. BIOS Final Report Nr. 986, S. 281 + 282).

Auch die Umsetzung von aminogruppengeschützten Aminobenzol-Verbindungen gemäß der Verfahrensweisen von BIOS Final Report Nr. 986, S. 285 - 288, der US-PS 2 459 002 und der europäischen Patentanmeldungs-Veröffentlichung Nr. 0 049 711A führen nicht zu Nitro-amino-benzol-Verbindungen, die in allen Fällen die erforderliche hohe Reinheit besitzen. So wird mit der erwähnten Verfahrensweise der EP-A-0 049 711 zwar eine Verbesserung des Verfahrens aus BIOS Final Report und aus US-PS 2 459 002 geltend gemacht (s. hierzu die Seiten 1 bis 3 und die Beispiele 17 und 18 sowie die Seiten 3 und 4 dieser EP-0 049 711A), jedoch wird auch in dieser Patentanmeldung auf Seite 35 angemerkt, daß erforderlichenfalls die nach dem Verfahren der EP-0 049 711 erhältlichen Nitro-aminobenzol-Verbindungen noch weiter durch Umkristallisation oder Sublimation oder Destillation gereinigt werden können. So genügt beispielsweise das nach Beispiel 1 dieser EP-0 049 711A erhaltene 5-Nitro-2-amino-1-methoxy-benzol nicht den Anforderungen der Praxis für die Verwendung als C.I. Azoic Diazo Compound 5.

Ähnlich mangelhaft sind auch die Herstellverfahren für 5-Nitro-2-amino-1-methyl-benzol (s. BIOS Final Report Nr. 986, S. 301, sowie Winnacker-Küchler, Chemische Technologie, 3. Auflage (1972), Band 4, S. 154).

Es bestand deshalb weiterhin ein Bedürfnis, Nitro-aminobenzol-Verbindungen in besserer Qualität zu erhalten.

Mit der vorliegenden Erfindung wurde nunmehr ein Verfahren zur Herstellung von reinen Nitro-aminobenzol-Verbindungen gefunden, das ohne zusätzliche Reinigungsverfahren auskommt und es ermöglicht, die Endprodukte direkt, d.h. ohne zusätzliche Reinigung, auch als Diazokomponenten zur Herstellung von Färbungen nach der Eisfarbentechnik einzusetzen.

Die vorliegende Erfindung betrifft ein Verfahren zur Herstellung von reinen Nitro-aminobenzol-Verbindungen, bei welchem man eine an der Aminogruppe mit einer üblichen Schutzgruppe versehenen Aminobenzol-Verbindung in üblicher Weise nitriert, die aminogruppengeschützte Nitro-aminobenzol-Verbindung anschließend isoliert und die Schutzgruppe mittels Schwefelsäure in üblicher Weise abspaltet, das dadurch gekennzeichnet ist, daß man das aus der Hydrolyse erhaltene Sulfatsalz der Nitro-aminobenzol-Verbindung in Form des reinen, kristallinen Sulfates aus der Reaktionsmischung abscheidet und isoliert und anschließend hieraus gewünschtenfalls die freie Nitro-aminobenzol-Verbindung in reiner Form gewinnt. Bevorzugt sind die Nitro-aminobenzol-Verbindungen 4-Nitro-1-aminobenzol- und 2-Nitro-1-aminobenzol-Verbindungen.

Die vorliegende Erfindung betrifft insbesondere ein Verfahren zur Reinigung von Nitro-aminobenzol-Verbindungen, das dadurch gekennzeichnet ist, daß man eine an der Aminogruppe mit einer Schutzgruppe versehenen Nitro-aminobenzol-Verbindung in wäßriger Schwefelsäure zur Nitro-aminobenzol-Verbindung

hydrolysiert, das Sulfatsalz der Nitro-aminobenzol-Verbindung aus dem Reaktionsgemisch abscheidet und isoliert und es anschließend gewünschtenfalls in die Nitro-aminobenzol-Verbindung auf üblichem Wege überführt.

Durch diese Verfahrensmaßnahmen wird die gewünschte Nitro-aminobenzol-Verbindung bzw. deren Sulfatsalz in für die Weiterverarbeitung in der Eisfarbentechnik genügend reiner Form erhalten.

Schutzgruppen sind im allgemeinen die üblichen Acylreste von aliphatischen oder aromatischen Carbonsäuren oder Sulfonsäuren, wie die von Alkancarbonsäuren von 2 bis 5 C-Atomen oder Alkansulfonsäuren von 1 bis 4 C-Atomen, wie insbesondere der Essigsäure, der Benzoesäure, der Methansulfonsäure, der Benzolsulfonsäure oder der p-Toluolsulfonsäure. Dementsprechend sind aminogruppengeschützte Nitro-aminobenzol- oder Aminobenzol-Verbindungen Nitro-acylamino-benzol- bzw. Acylamino-benzol-Verbindungen, bei welchen die Acylgruppe insbesondere ein Alkanoylrest von 2 bis 5 C-Atomen, ein Alkylsulfonylrest von 1 bis 4 C-Atomen, der Phenylsulfonylrest, der p-Toluylsulfonylrest, der Methylsulfonylrest oder der Benzoylrest ist.

Die Hydrolyse der Acylaminogruppe zur Aminogruppe im erfindungsgemäßen Verfahren wird in der Regel bei einer Temperatur zwischen 50 und 110 °C durchgeführt; bevorzugt erfolgt sie bei einer Temperatur zwischen 80 und 105 °C. Als wäßrige Schwefelsäure wird 30 bis 96 gew.-%ige Schwefelsäure, bevorzugt 40 bis 80 gew.-%ige Schwefelsäure verwendet, wobei die zu hydrolysierende Nitro-acylamino-benzol-Verbindung in der Regel in einer Konzentration (Menge) von 20 bis 100 Gew.-%, bevorzugt von 40 bis 90 Gew.-%, bezogen auf die wäßrige Schwefelsäure, in den Hydrolyseansatz eingesetzt wird (100 Gew.-% bspw. bedeutet also gleiche Teile Ausgangsprodukt und wäßrige Schwefelsäure). Die Schwefelsäurekonzentration soll hierbei so bemessen sein, daß eine gute Rührfähigkeit der Reaktionsmenge gewährleistet ist.

Nach beendeter Hydrolyse wird der Ansatz abgekühlt, wobei das Sulfatsalz des Nitro-aminobenzols sich bereits abscheiden kann. Um eine vollständige oder fast vollständige Auskristallisation des Nitro-aminobenzol-Sulfats zu gewährleisten, ist es vorteilhaft, die Schwefelsäurenkonzentration, sofern sie höher als 60 Gew.-% beträgt, durch Zugabe von Wasser oder verdünnter Schwefelsäure auf unterhalb 60 Gew.-% zu erniedrigen. Bevorzugt soll die Schwefelsäurenkonzentration nach Beendigung der Hydrolyse mittels Wasser oder verdünnter Schwefelsäure auf einen Konzentrationsbereich von 25 bis 50 Gew.-%, insbesondere von 30 bis 50 Gew.-%, eingestellt werden. Zu starke Verdünnungen sollten vermieden werden, um bei diesem Verfahrensschritt eine Hydrolyse des Sulfatsalzes zur freien Aminoverbindung auszuschließen. Das Wasser oder die verdünnte Schwefelsäure wird bevorzugt dem noch heißen Hydrolyseansatz zugesetzt.

Die günstigsten Konzentrationsbereiche der Schwefelsäure und des Gehaltes der Nitro-acylamino-benzol-Verbindung in den Reaktionsansätzen sowohl bei der Hydrolyse als auch insbesondere bei der Abscheidung des Nitro-aminobenzol-Sulfats sind jedoch im Einzelfall für jedes Produkt individuell zu ermitteln, was ohne Schwierigkeiten in einigen kleinen Vorversuchen durchgeführt werden kann. Hierbei sollten die Konzentrationen der Ausgangsverbindungen und der Schwefelsäure auch so gewählt werden, daß das Nitro-aminobenzol bei der Hydrolysetemperatur noch gelöst ist, um mit dem Hydrolyseansatz nach Abschluß der Hydrolyse erforderlichenfalls noch eine heiße Klärfiltration durchführen zu können.

Die aus der verdünnten wäßrigen Schwefelsäure isolierten Sulfatsalze der Nitro-aminobenzol-Verbindungen besitzen eine hohe Reinheit. Sie können direkt, sofern dies die Chemie der Weiterverarbeitung erlaubt, in Folgesynthesen eingesetzt werden; zum Beispiel können sie direkt als Entwicklungskomponenten (Diazokomponenten) zur Synthese von Azofarbstoffen auf der Faser gemäß der Eisfarbentechnik eingesetzt werden. Aus den reinen Sulfatsalzen lassen sich die freien Nitro-aminobenzol-Verbindungen auf üblichem Wege, so durch Behandlung mit Wasser oder mit einer ein alkalisch wirkendes Agenz, wie Natriumcarbonat oder Natriumhydroxid, enthaltenden wäßrigen Lösung und anschließende Isolierung, wie Filtration, gewinnen. Die hierbei entstehende wäßrige, verdünnte Schwefelsäure kann wiederum im erfindungsgemäßen Verfahren weiterverwendet werden, wie zum Einstellen der optimalen Säurekonzentration von konzentrierter Schwefelsäure für die Hydrolyse oder zum Verdünnen des Hydrolyseansatzes.

Bevorzugt lassen sich nach dem erfindungsgemäßen Verfahren solche Nitro-aminobenzol-Verbindungen reinigen, die den allgemeinen Formeln (1a) und (1b) entsprechen:

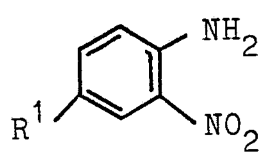

(1a)

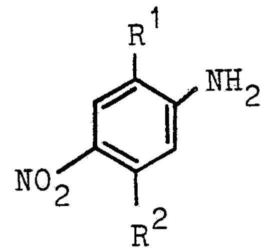

(1b)

in welchen

$R^1$ eine geradkettige oder verzweigte Alkylgruppe von 1 bis 3 C-Atomen oder eine geradkettige Alkoxygruppe von 1 bis 4 C-Atomen, insbesondere die Methyl-, Isopropyl-, Methoxy-, Ethoxy- oder n-Butoxy-Gruppe, und

$R^2$ ein Wasserstoffatom oder ein Halogenatom, wie Bromatom und insbesondere Chloratom, ist.

Dementsprechend dienen in der Hydrolysereaktion als Nitro-acylamino-benzol-Ausgangsverbindungen Verbindungen entsprechend den allgemeinen Formeln (2a) und (2b)

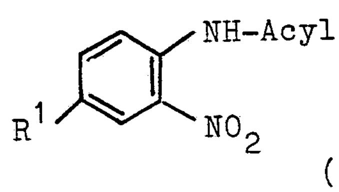

(2a)

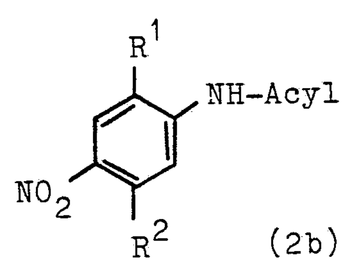

(2b)

in welchen $R^1$ und $R^2$ die obengenannten Bedeutungen haben und Acyl eine übliche Schutzgruppe, wie der Acylrest einer aliphatischen oder aromatischen Carbon- oder Sulfonsäure, wie insbesondere der Alkanoylrest von 2 bis 5 C-Atomen, hiervon bevorzugt der Acetylrest, oder der Benzoyl-, Phenylsulfonyl- oder p-Toloylsulfonyl-Rest, hiervon bevorzugt der Phenylsulfonylrest, oder eine Alkylsulfonylgruppe von 1 bis 4 C-Atomen, wie die Methylsulfonylgruppe, ist.

Die Ausgangsverbindungen der allgemeinen Formeln (2a) und (2b) können durch mehr oder weniger große Anteile an zu der jeweiligen Verbindung (2a) oder (2b) isomeren Verbindungen verunreinigt sein, wobei sich die isomeren Verbindungen insbesondere von den jeweilgen Verbindungen der Formeln (2) dadurch unterscheiden, daß die Nitrogruppe in eine andere als die in den Formeln (2a) bzw. (2b) angegebene Stellung besetzt.

Bevorzugt dient das Verfahren zur reinen Herstellung von 5-Nitro-2-amino-1-methyl-benzol, 5-Nitro-4-chlor-2-amino-1-methyl-benzol, 5-Nitro-2-amino-1-isopropyl-benzol und insbesondere 5-Nitro-2-amino-1-methoxy-benzol. Hierbei geht man bevorzugt von 5-Nitro-2-phenylsulfonylamino-1-methyl-benzol oder 5-Nitro-2-methylsulfonylamino-1-methyl-benzol, von 5-Nitro-4-chlor-2-phenylsulfonylamino-1-methyl-benzol oder 5-Nitro-4-chlor-2-methylsulfonylamino-1-methyl-benzol, von 5-Nitro-2-phenylsulfonylamino-1-isopropyl-benzol oder von 5-Nitro-2-methylsulfonylamino-1-isopropyl-benzol bzw. 5-Nitro-2-acetylamino-1-methoxy-benzol aus.

Insbesonders bevorzugt dient das erfindungsgemäße Verfahren zur reinen Herstellung von 5-Nitro-2-amino-1-methoxy-benzol, dessen Verunreinigung insbesondere die isomere 4-Nitro-2-amino-1-methoxy-benzol-Verbindung sein kann, indem man von deren entsprechenden 2-Acetylamino-Ausgangsverbindung-(en) ausgeht und es (sie) gemäß dem erfindungsgemäßen Verfahren der Schwefelsäurenhydrolyse unterwirft, das 5-Nitro-2-amino-1-methoxy-benzol als Sulfatsalz abscheidet und das Sulfat gewünschtenfalls in die freie Base überführt. Isomere Ausgangsgemische dieser Art erhält man aus Nitrierungsreaktionen von 2-Acetylamino-1-methoxy-benzol, wie sie beispielsweise aus der anfangs erwähnten Literatur bekannt sind.

Weiterhin dient das erfindungsgemäße Verfahren insbesondere zur Herstellung von reinem 3-Nitro-4-amino-1-methoxy-benzol, bevorzugt unter Verwendung von 3-Nitro-4-acetyl-amino-1-methoxy-benzol als Ausgangsverbindung.

Das erfindungsgemäße Verfahren hat nicht nur den großen Vorteil, daß man leicht direkt aus den Nitrierungsansätzen das isolierte Nitrierungsprodukt in wäßriger Schwefelsäure der gleichzeitigen Hydrolyse

und Reinigung unterwerfen kann, sondern auch den Vorteil, daß die nach Abtrennung der Nitro-aminobenzol-Sulfatsalze erhaltenen Mutterlaugen, die im allgemeinen relativ konzentrierte wäßrige Schwefelsäuren sind, mit einem wirtschaftlich vertretbaren Aufwand nach technisch bekannten Verfahren aufgearbeitet und regeneriert werden können, so daß - im Gegensatz zu den Herstellungsverfahren von Nitro-aminobenzol-Verbindungen nach dem Stand der Technik - keine zusätzliche Abwasserbelastung durch nicht regenerationsfähige wäßrige Schwefelsäure anfällt.

Die nachfolgenden Beispiele dienen zur näheren Bedeutung des Erfindungsgegenstandes. Teile sind Gewichtsteile und Prozentangaben beziehen sich auf Gewichtsprozente, sofern nicht anders vermerkt. Gewichtsteile stehen zu Volumenteilen im Verhältnis von Kilogrammm zu Liter.

**Beispiel 1:**

a) Unter Rühren gibt man in 650 Teile etwa 90 %ige wäßrige Schwefelsäure 308 Teile 5-Nitro-2-methyl-sulfonylamino-1-methoxy-benzol, das beispielsweise analog dem in BIOS Final Report Nr. 986, S. 272 oder S. 301, beschriebenen Verfahren erhältlich ist, erwärmt den Ansatz auf etwa 60 °C und führt die Umsetzung etwa 8 Stunden bei dieser Temperatur weiter. Anschließend gibt man langsam etwa 500 Teile Wasser hinzu und rührt den Ansatz bis zum Abkühlen auf etwa 20 °C, saugt das ausgeschiedene Sulfat des 5-Nitro-2-amino-1-methoxy-benzols ab und saugt es gut trocken.

Analyse einer von überschüssiger Schwefelsäure bspw. mit Aceton freigewaschenen und getrockneten Probe:

berechnet (auf $C_7H_{10}N_2O_7S$):
C 31,5 %, H 3,7 %, S 12,0 %;
gefunden:
C 31,2 %, H 3,7 %, S 12,4 %.

b) Zur Überführung des Sulfatsalzes in das freie 5-Nitro-2-amino-1-methoxy-benzol kann das aus der Hydrolysereaktion abgetrennte, schwefelsäure-feuchte Sulfatsalz mittels Wasser oder einer verdünnten wäßrigen Natronlauge behandelt werden. Dies kann auf dem Filter selbst erfolgen, oder man verrührt das Sulfatsalz in üblicher Weise mit Wasser oder der wäßrigen Natronlauge, saugt anschließend das ausgefallene Nitro-amino-methoxybenzol ab, wäscht es mit Wasser nach und trocknet es.

Ausbeute:

200 Teile entsprechend 95 % der Theorie, bezogen auf die unter a) eingesetzte Methylsulfonylamino-Verbindung.

Schmelzpunkt: 140-141 °C.

Die Verbindung ist ohne weitere Reinigung direkt als Diazokomponente (C.I. Azoic Diazo Compound 5) zur Herstellung von qualitativ hochwertigen Färbungen in der Eisfarbentechnik geeignet.

c) Die Mutterlauge, die nach Abtrennung des Sulfatsalzes erhalten wird, besteht im wesentlichen aus einem Gemisch von etwa 440 Teilen Schwefelsäure, 140 Teilen Methylschwefelsäure und etwa 600 Teilen Wasser. Sie kann ohne Vorbehandlung direkt zur Schwefelsäureregenevation, z. B. nach einem technisch üblichen thermischen Spaltverfahren, verwendet und aufgearbeitet werden.

**Beispiel 2:**

a) Unter Rühren hydrolysiert man 210 Teile eines isomeren Gemisches, bestehend aus 157 Teilen 5-Nitro-2-acetylamino-1-methoxy-benzol und 53 Teilen 4-Nitro-2-acetylamino-1-methoxy-benzol in 360 Teilen einer etwa 50 %igen wäßrigen Schwefelsäure bei einer Temperatur zwischen 80 und 85 °C während 3 bis 4 Stunden. Anschließend gibt man 200 Teile Wasser hinzu und klärt die Lösung mittels etwa 3 Teilen Aktivkohle und anschließende Filtration bei 80 bis 85 °C. Das Filtrat wird bis zur Abkühlung auf etwa 20 °C gerührt und aus ihm die abgeschiedenen beige-farbigen Kristalle des Sulfats von 5-Nitro-2-amino-1-methoxy-benzol abgetrennt. Die Kristalle können durch Nachwaschen bspw. mit Aceton von überschüssiger Schwefelsäure freigewaschen werden. Analyse:

ber. ($C_7H_{10}N_2O_7S$):
C 31,5 %, H 3,7 %, S 12,0 %;
gef.:
C31,0 %, H 3,8 %, S 12,5 %.

b) Sowohl das noch schwefelsäurehaltige als auch das von Schwefelsäure freigewaschene Sulfatsalz kann durch Verrühren in etwa 500 Teilen Wasser bei 25 °C zum 5-Nitro-2-amino-1-methoxy-benzol hydrolysiert werden, das in Form gelber Kristalle ausfällt. Nach Absaugen, Nachwaschen mit Wasser und Trocknen erhält man 108 Teile 5-Nitro-2-amino-1-methoxy-benzol mit einem Schmelzpunkt von 140,5-141 °C; die Ausbeute entspricht 86 % d.Th., bezogen auf den 5-Nitro-2-acetylamino-1-methoxy-benzol-Anteil im unter a) eingesetzten Isomerengemisch.

Die Verbindung kann ohne weitere Reinigung direkt als Diazokomponente zur Herstellung von qualitativ hochwertigen Färbungen gemäß der Eisfarbentechnik eingesetzt werden.

c) Die Mutterlauge, die nach Isolierung des Sulfatsalzes gemäß a) erhalten wird, versetzt man mit etwa 360 Teilen einer etwa 14 %igen wäßrigen Natronlauge. Das ausfallende Produkt besteht aus etwa 20 Teilen eines 1:1-Gemisches von 5-Nitro- und 4-Nitro-2-amino-1-methoxy-benzol, das dem nächsten Hydrolysenansatz zugesetzt werden kann.

Aus der nach dieser Fällung des Isomerengemisches erhaltenen Mutterlauge können mittels Natriumchlorid etwa 42 Teile 4-Nitro-2-amino-1-methoxy-benzol als Hydrochlorid mit einem Reingehalt von etwa 90 % ausgesalzen werden; die restlichen 10 % des ausgesalzenen Produktes bestehen im wesentlichen aus Natriumchlorid und Natriumsulfat. Das Hydrochlorid kann als Diazokomponente C.I. Azoic Diazo Compound 13 für die Herstellung von qualitativ hochwertigen Färbungen in der Eisfarbentechnik eingesetzt werden. Aus ihm gewinnt man durch Behandlung mit verdünnter Natronlauge das freie 5-Nitro-2-amino-1-methoxy-benzol mit einem Schmelzpunkt von 116-117 °C.

**Beispiel 3:**

224 Teile eines Isomerengemisches, bestehend aus 5-Nitro-2-propionylamino-1-methoxy-benzol und 4-Nitro-1-propionyl-amino-1-methoxybenzol, das durch Nitrierung von 2-Propionylamino-1-methoxy-benzol gemäß der Verfahrensweise von BIOS Final Report Nr. 986, S. 297, erhältlich ist, wird entsprechend den Angaben des Beispieles 2 in wäßriger Schwefelsäure hydrolysiert. Die Aufarbeitung des erfindungsgemäßen Ansatzes erfolgt gemäß den Angaben des Beispieles 2.

Es werden 127 Teile 5-Nitro-2-amino-1-methoxy-benzol (Smp.: 140 °C) und 17 Teile 4-Nitro-2-amino-1-methoxy-benzol (Smp.: 116 °C) in reiner Form erhalten, so daß diese Verbindungen jeweils als Diazokomponenten zur Herstellung von qualitativ hochwertigen Färbungen gemäß der Eisfarbentechnik eingesetzt werden können.

**Beispiel 4:**

75,6 Teile eines Isomerengemisches aus 5-Nitro-2-acetylamino-1-n-butoxy-benzol und 4-Nitro-2-acetylamino-1-n-butoxy-benzol, das aus der Nitrierung von 2-Acetylamino-1-n-butoxy-benzol gemäß der Verfahrensweise von BIOS Final Report Nr. 986, S. 279, erhältlich ist, wird unter Rühren in 160 Teilen einer etwa 50 %igen wäßrigen Schwefelsäure bei einer Temperatur zwischen 80 und 85 °C während 3 Stunden hydrolysiert. Anschließend gibt man etwa 60 Teile Wasser hinzu und kühlt den verdünnten Ansatz unter Rühren auf etwa 20 °C ab. Das ausgeschiedene 5-Nitro-2-amino-1-n-butoxy-benzol-Sulfat wird abgesaugt und durch Verrühren mit etwa 200 Teilen Wasser zur freien Aminobenzol-Verbindung hydrolysiert. Die feste Verbindung wird vom Wasser abgetrennt, mit etwas Wasser nachgewaschen und getrocknet.

Man erhält etwa 33 Teile reines 5-Nitro-2-amino-1-n-butoxy-benzol mit einem Schmelzpunkt von 68-70 °C.

Aus dem Filtrat kann man durch Neutralisation mit wäßriger Natronlauge etwa 11 Teile 4-Nitro-2-amino-1-n-butoxy-benzol mit einem Schmelzpunkt von 55 °C abscheiden.

**Beispiel 5:**

Man rührt 210 Teile technisches 4-Acetylamino-3-nitro-1-methoxy-benzol während 3 Stunden in 360 Teilen einer etwa 80-85 °C heißen 50 %igen wäßrigen Schwefelsäure, rührt sodann dan Ansatz unter Abkühlen auf etwa 20 °C weiter und filtriert vom ausgeschiedenen Sulfatsalz der Aminobenzol-Verbindung ab. Eine Probe des schwefelsäurefrei gewaschenen und getrockneten Sulfats zeigt folgende Analysenwerte:

ber. ($C_7H_{10}N_2O_7S$):

C 31,6 %, H 3,8 %, S 12,0 %;

gef.:

C 30,5 %, H 3,7 %, S 12,5 %.

Das Sulfatsalz wird mit verdünnter wäßriger Natronlauge in üblicher Weise in die freie Aminobenzol-Verbindung übergeführt.

Ausbeute:

153 Teile 4-Amino-3-nitro-methoxy-benzol (entspr. 90 % d.TH.).

Es kann ohne weitere Reinigung als Diazokomponente C.I. Azoic Diazo Compound 1 zur Herstellung von qualitativ hochwertigen Färbungen gemäß der Eisfarbentechnik eingesetzt werden.

**Beispiel 6:**

241 Teile eines wasserfeuchten, technischen, aus einer Nitrierungsreaktion erhaltenen Produktes mit einem Gehalt von etwa 91 % 5-Nitro-2-phenylsulfonylamino-1-methyl-benzol werden unter Rühren bei einer Temperatur von 85-90 °C während 4 bis 5 Stunden in 242 Teilen einer 95 %igen wäßrigen Schwefelsäure hydrolysiert. Anschließend gibt man langsam unter Rühren 120 Teile Wasser hinzu, rührt bis zum Abkühlen auf etwa 20 °C nach und filtriert das ausgeschiedene 5-Nitro-2-amino-1-methyl-benzol-Sulfat ab. Eine - schwefelsäurefrei gewaschene und getrocknete Probe zeigt folgende Analysenwerte:

ber. ($C_7H_{10}N_2O_6S$):

C 33,7 %, H 4,0 %, N 11, 2 %, S 12,8 %;

gef.:

C 33,1 %, H 4,0 %, N 11,2 %, S 13,0 %.

Dieses Sulfatsalz ist ausreichend rein, um direkt als Diazokomponete zur Herstellung von qualitativ hochwertigen Färbungen gemäß der Eisfarbentechnik verwendet zu werden.

Aus dem Sulfat kann durch Verrühren mit Wasser oder wäßrigem Alkali in üblicher Weise die freie Aminobenzol-Verbindung gewonnen werden. Man erhält 108 Teile 5-Nitro-2-amino-1-methyl-benzol in einer Ausbeute von 95 % d.Th. mit einem Schmelzpunkt von 132 - 132,5 °C. Sie kann ebenso direkt als Diazokomponente C.I. Azoic Diazo Compound 34 in Eisfarbenfärberei eingesetzt werden.

Das schwefelsaure Filtrat enthält etwa 50 % Schwefelsäure, etwa 33 % Benzolsulfonsäure und etwa 17 % Wasser; es kann ohne weitere Vorbehandlung einer üblichen Schwefelsäureregeneration, beispielsweise nach dem Spaltverfahren, zugeführt werden.

**Beispiel 7:**

Man verrührt 271 Teile eines wasserfeuchten, technischen, aus einer Nitrierungsreaktion erhaltenen Produktes mit einem Gehalt von etwa 85 % 5-Nitro-2-methylsulfonylamino-1-methyl-benzol mit 323 Teilen einer 95 %igen Schwefelsäure, erwärmt es auf 100 °C und führt die Hydrolyse während 4 bis 5 Stunden bei dieser Temperatur unter Rühren weiter. Anschließend gibt man langsam bei etwa 70 °C 140 Teile Wasser hinzu, läßt sodann unter Rühren auf etwa 20 °C abkühlen und isoliert das abgeschiedene Sulfatsalz des 5-Nitro-2-amino-1-methyl-benzols.

Eine Probe des schwefelsäurefrei gewaschenen und getrockneten Sulfats zeigt folgende Analysenwerte (bezogen auf $C_7H_{10}N_2O_6S$):

ber.:

C 33,7 %, H 4,0 %, S 12,8 %;

gef.:

C 33,0 %, H 3,8 %, S 13,2 %.

Das Sulfatsalz besitzt eine genügend hohe Reinheit, um direkt als Diazokomponente zur Herstellung von qualitativ hochwertigen Färbungen gemäß der Eisfarbentechnik eingesetzt werden zu können.

Mit verdünnter wäßriger Natronlauge kann in üblicher Weise das Sulfat in die freie Aminobenzol-Verbindung überführt werden. Man erhält 146 Teile 5-Nitro-2-amino-1-methyl-benzol mit einem Schmelzpunkt von 132-132,5 °C. Die Reinheit dieser Aminobenzol-Verbindung gewährleistet die direkte Verwendung als Diazokomponente C.I. Azoic Diazo Compound 34.

Die aus der Sulfatisolierung erhältliche salzfreie Mutterlauge besteht aus etwa 60 % Schwefelsäure, etwa 20 % Methylschwefelsäure und etwa 20 % Wasser. Sie kann gemäß den üblichen technischen Verfahren, wie einem Schwefelsäure-Spaltprozeß, zugeführt werden.

**Beispiel 8:**

147 Teile eines wasserfeuchten Nitrierungsproduktes mit einem Gehalt von etwa 90 % an 4-Chlor-5-nitro-2-methyl-sulfonylamino1-methyl-benzol wird unter Rühren in 250 Teilen einer etwa 95 %igen Schwefelsäure während 6 Stunden bei 100 °C hydrolysiert. Anschließend gibt man bei etwa 70 °C langsam 120 Teile Wasser hinzu und trennt nach Abkühlen auf 20 °C das abgeschiedene Sulfat des 4-Chlor-5-nitro-2-amino-1-methyl-benzols ab.
Eine schwefelsäurefrei gewaschene und getrocknete Probe zeigt folgende Analysenwerte (bezogen auf $C_7H_9ClN_2O_6S$):

ber.:
C 29,6 %, H 3,2 %, S 11,3 %;
gef.:
C 29,0 %, H 3,1 %, S 11,8 %.

Mit verdünnter wäßriger Natronlauge wird in üblicher Weise das Sulfat in die freie Aminobenzol-Verbindung überführt. Man erhält etwa 85 Teile 4-Chlor-5-nitro-2-amino-1-methyl-benzol mit einem Schmelzpunkt von 167 °C (entspr. 92 % d.Th., bezogen auf 4-Chlor-5-nitro-2-methyl-sulfonylamino-1-methyl-benzol).

**Beispiel 9:**

Man verrührt 71 Teile eines wasserfeuchten, aus einer Nitrierungsreaktion abgetrennten Produktes mit einem Gehalt von etwa 90 % an 5-Nitro-2-methylsulfonylamino-1-isopropyl-benzol in 81 Teilen einer 95 %igen Schwefelsäure, erwärmt den Ansatz von 90-100 °C und führt die Hydrolyse unter Rühren während 5 Stunden innerhalb dieses Temperaturbereiches durch. Anschließend gibt man langsam etwa 40 Teile Wasser hinzu, läßt den Ansatz auf etwas 20 °C abkühlen und isoliert das ausgeschiedene Sulfat des 5-Nitro-2-amino-1-isopropyl-benzols.
Eine Probe des schwefelsäurefrei gewaschenen und getrockneten Sulfats gibt folgende Analysenwerte (bezogen auf $C_9H_{14}N_2O_6S$):

ber.:
C 38,8 %, H 5,0 %, S 11,5 %;
gef.:
C 37,0 %, H 5,2 %, S 12,3 %.

Das Sulfatsalz wird in üblicher Weise mit wäßriger Natronlauge in die freie Aminobenzol-Verbindung überführt. Man erhält 42 Teile 5-Nitro-2-amino-1-isopropyl-benzol mit einem Schmelzpunkt von 51 - 53 °C (Ausbeute: 93 % d. Th., bezogen auf das 5-Nitro-2-methylsulfonylamino-1-isopropyl-benzol).
Es besitzt folgende Analysenwerte (bezogen auf $C_9H_{12}N_2O_2$):

ber.:
C 60,0 %, H 6,7 %, N 15,6 %;
gef.:
C 60,1 %, H 6,7 %, N 15,5 %.

Die so erhaltene Aminobenzol-Verbindung kann sowohl als freies Amin als auch als Sulfatsalz direkt als Diazokomponente zur Herstellung von qualitativ hochwertigen Färbungen gemäß der Eisfarbtechnik verwendet werden.

**Beispiel 10:**

130 Teile eines wasserfeuchten Produktes mit einem Gehalt von etwa 80 % an 3-Nitro-4-methylsulfonylamino-1-isopropyl-benzol werden bei einer Temperatur zwischen 90 und 100 °C während etwa 6 Stunden unter Rühren in 130 Teilen einer etwa 95 %igen wäßrigen Schwefelsäure hydrolysiert. Anschließend gibt man langsam 100 Teile Wasser hinzu und läßt den Ansatz danach unter Rühren auf etwa 20 °C abkühlen, filtriert vom ausgeschiedenen Sulfat des 3-Nitro-4-amino-1-isopropyl-benzols ab und überführt das Sulfatsalz durch Verrühren mit Wasser bei etwa 25 °C oder mittels verdünnter wäßriger Natronlauge in die freie Aminobenzol-Verbindung.

Das reine 3-Nitro-4-amino-1-isopropyl-benzol wird in einer Ausbeute von 93 % d.Th. (65 Teile) mit einem Schmelzpunkt von 36-37 °C erhalten. Es geht direkt zum Einsatz als Diazokomponente zur Herstellung von qualitativ hochwertigen Färbungen gemäß der Eisfarbentechnik.

**Ansprüche**

1. Verfahren zur Herstellung einer reinen Nitro-aminobenzol-Verbindung, bei welchem man eine an der Aminogruppe mit einer Schutzgruppe versehenen Aminobenzol-Verbindung in üblicher Weise nitriert, die aminogruppengeschützte Nitro-aminobenzol-Verbindung anschließend isoliert und die Schutzgruppe mittels Schwefelsäure in üblicher Weise abspaltet, dadurch gekennzeichnet, daß man das aus der Hydrolyse erhaltene Sulfatsalz der Nitro-aminobenzol-Verbindung in Form des reinen, kristallinen Sulfates aus der Reaktionsmischung abscheidet und isoliert und hieraus gewünschtenfalls die freie Nitro-aminobenzol-Verbindung in reiner Form gewinnt.

2. Verfahren nach Anspruch 1 zur Reinigung einer Nitro-aminobenzol-Verbindung, dadurch gekennzeichnet, daß man eine an der Aminogruppe mit einer Schutzgruppe versehene Nitro-aminobenzol-Verbindung in wäßriger Schwefelsäure zur Nitro-aminobenzol-Verbindung hydrolysiert, das Sulfatsalz der Nitro-aminobenzol-Verbindung aus dem Reaktionsgemisch abscheidet und isoliert und es anschließend gewünschtenfalls in die Nitro-aminobenzol-Verbindung überführt.

3. Verfahren nach Anspruch 1 oder 2, dadurch gekennzeichnet, daß die Hydrolyse der mit der Schutzgruppe versehenen Aminogruppe bei einer Temperatur zwischen 50 und 110 °C durchgeführt wird.

4. Verfahren nach Anspruch 1 oder 2, dadurch gekennzeichnet, daß die Hydrolyse der mit der Schutzgruppe versehenen Aminogruppe bei einer Temperatur zwischen 80 und 105 °C durchgeführt wird.

5. Verfahren nach mindestens einem der Ansprüche 1 bis 4, dadurch gekennzeichnet, daß die Umsetzung in einer 30 bis 95 gew.-%igen Schwefelsäure durchgeführt wird.

6. Verfahren nach mindestens einem der Ansprüche 1 bis 4, dadurch gekennzeichnet, daß die Hydrolyse in einer 40 bis 70 gew.-%igen Schwefelsäure durchgeführt wird.

7. Verfahren nach mindestens einem der Ansprüche 1 bis 6, dadurch gekennzeichnet, daß die zu hydrolysierende aminogruppengeschützte Nitro-aminobenzol-Verbindung im schwefelsauren Reaktionsansatz in einer Konzentration von 20 bis 100 Gew.-%, bezogen auf die eingesetzte wäßrige Schwefelsäure, vorliegt.

8. Verfahren nach mindestens einem der Ansprüche 1 bis 7, dadurch gekennzeichnet, daß man die Abscheidung der hydrolysierten Nitro-aminobenzol-Verbindung als Sulfatsalz aus einer wäßrigen Schwefelsäure mit einer Schwefelsäurekonzentration von 25 bis 50 Gew.-% vornimmt.

9. Verfahren nach mindestens einem der Ansprüche 1 bis 8, dadurch gekennzeichnet, daß die zur reinigende Nitro-aminobenzol-Verbindung eine Verbindung der allgemeinen Formal (1a) oder (1b)

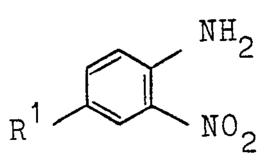
(1a)

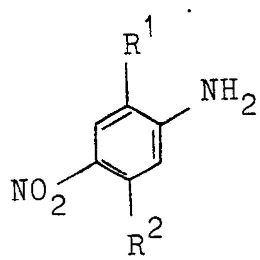
(1b)

ist, in welchen

$R^1$ eine geradkettige oder verzweigte Alkylgruppe von 1 bis 3 C-Atomen oder eine geradkettige Alkoxygruppe von 1 bis 4 C-Atomen und

$R^2$ ein Wasserstoffatom oder ein Halogenatom ist, und man von deren entsprechenden Ausgangsverbindung der allgemeinen Formel (2a) oder (2b)

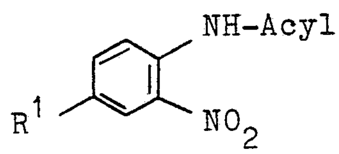

(2a)

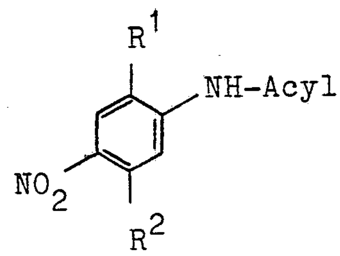

(2b)

ausgeht, in welchen $R^1$ und $R^2$ die obengenannten Bedeutungen haben und Acyl der Acylrest einer aliphatischen oder aromatischen Carbon- oder Sulfonsäure ist.

10. Verfahren nach mindestens einem der Ansprüche 1 bis 9, dadurch gekennzeichnet, daß die zu reinigende Verbindung entsprechend der allgemeinen Formel (1a) durch eine bezüglich der Nitrogruppe isomere Verbindung bzw. deren Acylamino-Derivat verunreinigt ist.

11. Verfahren nach mindestens einem der Ansprüche 1 bis 9, dadurch gekennzeichnet, daß die zu reinigende Verbindung entsprechend der allgemeinen Formel (1b) durch eine bezüglich der Nitrogruppe isomere Verbindung bzw. deren Acylamino-Derivat verunreinigt ist.

12. Verfahren nach mindestens einem der Ansprüche 1 bis 11, dadurch gekennzeichnet, daß die zu reinigende Nitro-aminobenzol-Verbindung das 5-Nitro-2-amino-1-methyl-benzol, 5-Nitro-4-chlor-2-amino-1-methyl-benzol oder 5-Nitro-2-amino-1-isopropyl-benzol ist.

13. Verfahren nach mindestens einem der Ansprüche 1 bis 9 und 11, dadurch gekennzeichnet, daß die zu reinigende Nitro-aminobenzol-Verbindung das 5-Nitro-2-amino-1-methoxy-benzol ist.

14. Verfahren nach mindestens einem der Ansprüche 1 bis 10, dadurch gekennzeichnet, daß die zu reinigende Nitro-aminobenzol-Verbindung das 3-Nitro-4-amino-1-methoxy-benzol ist.

15. Verfahren nach Anspruch 13, dadurch gekennzeichnet, daß das zu reinigende 5-Nitro-2-amino-2-methoxy-benzol bzw. dessen Acylamino-Derivat durch deren isomeres 4-Nitro-2-amino-1-methoxy-benzol bzw. durch dessen Acylamino-Derivat verunreinigt ist.